(19) Europäisches Patentamt
European Patent Office
Office européen des brevets

(11) **EP 1 016 384 B1**

(12) **EUROPEAN PATENT SPECIFICATION**

(45) Date of publication and mention
of the grant of the patent:
**13.08.2008  Bulletin 2008/33**

(51) Int Cl.:
***A61F 2/06*** (2006.01)

(21) Application number: **98941757.1**

(86) International application number:
**PCT/JP1998/004015**

(22) Date of filing: **08.09.1998**

(87) International publication number:
**WO 1999/012496 (18.03.1999 Gazette 1999/11)**

(54) **BLOOD VESSEL PROSTHESIS**

BLUTGEFÄSSPROTHESE

PROTHESE DE VAISSEAU SANGUIN

(84) Designated Contracting States:
**BE DE ES FR GB IT NL SE**

(30) Priority: **08.09.1997   JP 24321497**

(43) Date of publication of application:
**05.07.2000   Bulletin 2000/27**

(73) Proprietor: **TERUMO KABUSHIKI KAISHA**
**Tokyo 151-0072 (JP)**

(72) Inventor: **KOBAYASHI, Fumiaki,**
**Terumo Kabushiki Kaisha**
**Kanagawa 259-0151 (JP)**

(74) Representative: **Gillard, Marie-Louise et al**
**Cabinet Beau de Loménie**
**158, rue de l'Université**
**75340 Paris Cédex 07 (FR)**

(56) References cited:
**EP-A- 0 157 178          JP-A- 8 332 218**
**JP-A- 61 185 271        US-A- 5 354 329**

## Description

TECHNICAL FIELD

The present invention relates to a blood vessel prosthesis to be used in substituting or by-passing diseased in blood vessels.

BACKGROUND ART

In vascular diseases such as arteriosclerosis obliterans, aortic aneurysm, and aortic dissection, there have frequently been performed operations for substituting or by-passing diseased vessel with artificial blood vessel prosthesis. In such cases, those blood vessel prostheses have been used that are porous and facilitate the penetration of cells and the like from the point of view that desirably they merge with the host tissue quickly after the implantation and acquire antithromboticity or resistance to infection that the host tissue have.

Conventionally, when implanting such a porous blood vessel prosthesis, it has been a general measure to perform preclotting (operation of preliminarily clotting a prosthesis with albumin or blood of the patient himself) for the purpose of preventing the leakage of blood from the prosthesis. Recently, a blood vessel prosthesis material called "sealed graft" that consists of a conventional porous prosthesis as a base material sealed with a biologically absorbable material such as gelatin or collagen, thus requiring no preclotting any longer, has made an entry into the market and has been used widely.

However, as to the sealed graft, nobody denies the advantage that preclotting is unnecessary while they have pointed out various problems such as blood leakage due to uneven coverage by the sealing material or its detachment during implantation operation, bleeding from a needle hole in the anastomotic part, sustained fever and retention of exudates after implantation. As the measure for solving the above problems of the conventional sealed graft while retaining the advantage that preclotting is uncessary, it is effective to use a biologically non-absorbable material, particularly an elastomer material, as a sealing material as disclosed in, for example, JP-A-5-131025 and JP-A-7-51354. On the contrary, this has a difficulty in merging with the host tissue since the sealing material cannot be replaced with the host tissue.

In this regard, the blood vessel prosthesis of a laminate structure composed of an inner ply and an outer ply, each being porous, and a nonporous intermediate ply as disclosed in U.S. Patent No. 4, 743, 252, JP-A-8-332218 and US-A-5354329 requires no preclotting also and realizes merge with the tissue of organism while solving the problems of the conventional sealed graft.

However, the blood vessel prosthesis disclosed in U.S. Patent 4,743,252 has an outer ply that is formed by such as extruding a high molecular spinning solution onto a rotating mandrel and winding it therearound. It has the defects that when it is cut to a length suitable for use the blood vessel prosthesis it tends to fray, is difficult to control its porosity and the like, has a low mechanical strength, and so on.

The blood vessel prosthesis disclosed in JP-A-8-332218 has an outer ply that is preferably formed by subjecting nonwoven fabric to heat treatment so that it can be wound. The blood vessel prosthesis is free of the problem of fraying as observed in the blood vessel prosthesis described in the above U.S. patent. However, apart from its application to small blood vessels for which reinforcement by the surrounding tissues can be expected, for applying it to thoracic or abdominal aorta, the problem in strength has remained to be solved.

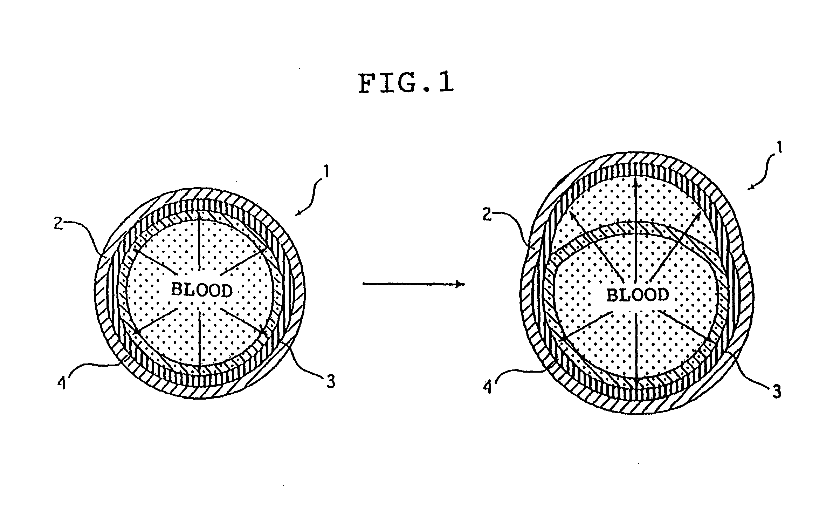

Referring to Fig. 1, more concrete explanation will be made. When use is made of a blood vessel prosthesis 1 having an outer ply 2 and an inner ply 4, each being porous, and a nonporous intermediate ply 3, blood passes through the inner ply 4, which is porous, to reach the nonporous intermediate ply 3. As a result, a separation force is generated between the inner ply 4 and the intermediate ply 3 and once separation occurs between the inner ply 4 and the intermediate ply 3, the function of the inner ply material 4 that has thus far indirectly supported blood through intermediary of adhesion to the inner ply material 3 is lost, resulting in that the force applied to the intermediate ply material 3 and the outer ply material 2 increases abruptly (Fig. 1). When the blood vessel prosthesis 1 is applied to small blood vessels, it can be expected that the surrounding tissues penetrate into the porous outer ply 2 to increase its strength or the surrounding tissues adhere on the outer side of the outer ply 2 to support it. Therefore, there is no problem. However, in the thoracic or abdominal cavity where no such reinforcing effect by the surrounding tissues can be expected, the blood vessel prosthesis cannot be applied since there is the fear of the occurrence of inter-ply separation between the inner ply 4 and the intermediate ply 3 or further of the breakage of the blood vessel prosthesis or diastasis of the anastomotic part.

Therefore, there is desired a blood vessel prosthesis of a laminate structure composed of an inner ply and an outer ply, each being porous, and a nonporous intermediate ply and having a strength characteristic such that it can be implanted into thoracic or abdominal cavity where no reinforcing effect by the surrounding tissues can be expected. However, there has hitherto been no such blood vessel prosthesis.

An object of the present invention is to provide a blood vessel prosthesis that requires no preclotting, merges with the tissue of organism quickly after the implantation and can be applied to thoracic or abdominal aorta safely.

DISCLOSURE OF THE INVENTION

As a result of intensive study, the present inventors have found that the above problems can be solved by controlling the elongation properties of the blood vessel prosthesis and its outer ply material within a certain relationship, thus achieving the present invention.

The present invention relates to a tubular blood vessel prosthesis composed of an outer ply and an inner ply, each being biologically non-absorbable and porous, and a biologically non-absorbable and nonporous intermediate ply, the blood vessel prosthesis satisfying the relationships of 1.2a+20≧b and a≦40 when a pressure of 200 mmHg is applied to the inner cavity of itself and the inner cavity of its outer ply, wherein a and b refer to percentage increases in the inner diameters of the above cavities, respectively.

According to one preferred embodiment, the inner diameter of the blood vessel prosthesis is 14-40 mm.

It is preferred that the inner ply and/or outer ply be composed of knitted or woven texture of crimpy fiber. More preferably, the crimpy fiber be conjugated fiber of polyethylene terephthalate and polybutylene terephthalate.

It is preferred that the inner ply and/or outer ply be composed of knitted or woven texture of shrunk fiber having a boiling water shrink ratio of 15 to 75%.

Also, the present invention relates to a blood vessel prosthesis composed of a porous outer ply, a substantially nonporous intermediate ply and a porous inner ply, each ply being composed of a biologically non-absorbable material, the blood vessel prosthesis satisfying the relationships of (1.2a+20)>b when the blood vessel prosthesis is drawn at a force of 50 gf ($5\times10^{-3}\times9.8$N) or less per mm of width, wherein a and b refer to percentages of elongation of the blood vessel prosthesis itself and the outer ply element alone, respectively.

It is preferred that the above inner ply be a construct obtained by knitting, weaving or braiding crimpy fiber. More preferably, the crimpy fiber is a conjugated fiber of polyethylene terephthalate and polybutylene terephthalate.

The outer ply is preferably a construct obtained by knitting, weaving or braiding polyester fiber.

Further, the prsent invention provides a method for producing the blood vessel prosthesis, characterized by providing an intermediate ply material on a surface of an inner ply material, covering thereon an outer ply material consisting of crimpy fiber or shrunk fiber, then heating the outer ply material to shrink so that it can closely adhere to the intermediate ply, and simultaneously or thereafter heating the intermediate ply material to melt it to thereby heat bonding the inner ply material, intermediate ply material and outer ply material.

Also, the present invention provides a method for producing the blood vessel prosthesis, characterized by providing an intermediate ply material on a surface of an outer ply material, covering thereon an inner ply material consisting of crimpy fiber or shrink fiber, heating the inner ply material to shrink it so that it can closely adhere to the intermediate ply, simultaneously or thereafter heating the intermediate ply material to melt it to thereby heat bonding the inner ply material, intermediate ply material, and outer ply material, and reversing the tube inside out.

BRIEF DESCRIPTION OF THE DRAWINGS

Fig. 1 is a diagram showing a blood vessel prosthesis of a laminate structure composed of an inner ply and an outer ply, each being porous, and a nonporous intermediate ply in a state where separation has occurred between the inner ply and the intermediate ply.

Fig. 2 is a cross-sectional view showing the blood vessel prosthesis of the present invention.

BEST MODE FOR CARRYING OUT THE INVENTION

Hereafter, the present invention will be described in detail.

The present invention relates to a tubular blood vessel prosthesis composed of an outer ply and an inner ply, each being biologically nonabsorbable and porous, and an intermediate ply which is biologically non-absorbable and nonporous.

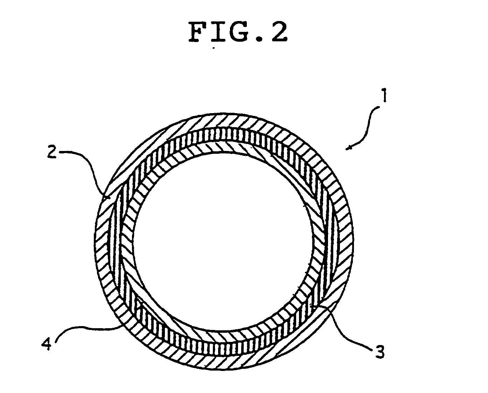

Referring to Fig. 2, an embodiment of the blood vessel prosthesis of the present invention will be explained. Fig. 2 is a cross-sectional view of the blood vessel prosthesis 1 of the present invention.

A blood vessel prosthesis 1 is composed of a porous outer ply 2, a substantially nonporous intermediate ply 3, and a porous inner ply 4 in order from the outside. In the present invention, the blood vessel prosthesis may be provided with other plies between the outer ply and the intermediate ply and between the intermediate ply and the inner ply, respectively. Furthermore, each ply may be formed of a multi-layer structure.

The outer ply 2 and the inner ply 4 are not particularly limited so long as each of them is biologically non-absorbable and porous materials. As will be later on, the outer ply 2 and the inner ply 4 require different physical properties and it is preferred that appropriate materials be selected and used, respectively. Also, when the same material is selected for both the outer ply 2 and the inner ply 4, the degrees of crimp or of knitting or weaving may be varied such that the each requirement of physical properties can be satisfied.

As the biologically non-absorbable material used for the outer ply 2 and the inner ply 4, polyester fibers can be used advantageously taking into consideration actual results of use, mechanical strength and the like. Example of the polyester fiber include polyethylene terephthalate, polybutylene terephthalate, polyester-polyether block copolymer, polyester-polyester block copolymer and composite fibers thereof.

The inner ply 4 may be made of a material that readily elongates by a small force. For example, there can be used resilient materials such as silicone rubber, styrene-based elastomer, and olefin-based elastomer. Specifically, the styrene-based elastomer includes Crayton, Kaliflex produced by Shell Chemical Co.; Tafprene, Taftech produced by Asahi Kasei Chemical Industry Co., Aron AR produced by Aron Kasei Co.; Ravalon produced by Mitsubishi Chemical Co.; JSR-TR, JSR-SIS, Dynalon produced by JSR Corp.; and Septon produced by Kuraray Co. The olefin-based elastomer includes Milastomer, Tafmer produced by Mitsui Chemical Co.; Sumitomo TPE produced by Sumitomo Chemical Industry Co.; and Thermolan produced by Mitsubishi Petrochemical Co.

The porous material is preferably of a knitted or woven texture in light of ease of penetration of the tissue of organism and strength properties. In the knitted or woven textures, crimpy fiber or shrunk fiber can be used advantageously. This is because crimpy fiber or shrunk fiber allows use of the production method described hereinbelow.

As the crimpy fiber may be used known ones such as bonded type (two or more materials are bonded together), core-sheath type (one component is used as a core and other component or components surround it). Of these, a conjugated fiber of bonding type of polyethylene terephthalate and polybutylene terephthalate is preferable. As the shrunk fiber may be used those known that are made by melt extruding a polymer thorough a nozzle, winding it at a high speed, drawing it at a low temperature. Among those, shrunk fiber composed of polyethylene terephthalate having a boiling water shrink ratio of 15% or more. In the present invention, the upper limit of the boiling water shrink ratio is not particularly limited and those having a boiling water shrink ratio of about 75%, which is the general upper limit of the shrunk fiber obtained by the known method, can be used.

The intermediate ply 3 is not particularly limited so long as it is biologically non-absorbable and nonporous. However, it is preferred that it be made of a material that readily elongates by a small force and having the function of suppressing bleeding through the needle hole.

The biologically non-absorbable material used in the intermediate ply 3 includes, for example, resilient materials such as silicone rubber, styrene-ethylenebutylene-styrene (SEBS) block copolymer, styrene-ethylenepropylene-styrene (SEPS) block copolymer, and linear low density polyethylene (L-LDPE). Among these, thermoplastic elastomers containing no unsaturated bond, such as SEBS and SEPS, are stable in the organism and allows use of production method that employs thermal fusion without adhesive or solvent upon bonding the inner ply and outer ply. In this regard, such thermoplastic elastomers are particularly suitable as a material for the intermediate ply of the present invention. These materials may be used after adding additives such as plasticizers, if required.

As the intermediate ply 3 may be used a material having blended therein an isoprene derivative in an amount of 20 parts by weight or more per 100 parts by weight of styrene-based elastomer and/or olefin-based elastomer. The isoprene derivative includes, for example, squalane, squalene and the like. These may be used advantageousy regardless of whether they are natural substance or derivatives thereof, or chemically synthesized ones.

The intermediate ply 3 may be made substantially nonporous by use of the above biologically nonabsorbable material.

One of the features of the blood vessel prosthesis of the present invention is that the blood vessel prosthesis satisfies the relationships of $1.2a+20 \geq b$ and $a \leq 40$ when a pressure of 200 mmHg ($200 \times 13.6 \times 980.7 \times 10^{-2}$ N·m$^{-2}$) is applied to the inner cavity of itself and the inner cavity of its outer ply, wherein a and b refer to percentage increases in the inner diameters of the above cavities, respectively.

The states in which a pressure of 200 mmHg is applied to the inner cavity of the blood vessel prosthesis and the inner cavity of the outer ply simulate the state in which the blood vessel prosthesis is clinically used normally and the state in which the inner ply and the intermediate ply are separated from each other with concomitant breakage of the intermediate ply. Therefore, the blood vessel prosthesis and its outer ply material of the present invention must not be broken by the pressure of at least 200 mmHg.

The blood vessel prosthesis can be directly pressurized by inclusion of air or water in the cavity thereof. The outer ply is porous so that similar pressurization methods cannot be adopted. Therefore, for example, an indirect pressurization method such as insertion of a rubber tube into the cavity may be used. That is, the pressure P mmHg necessary for inflating the tube is measured in advance and pressurizing of (200+P) mmHg the rubber tube inserted into the cavity of outer ply is adjusted so that the pressure of exactly 200 mmHg is applied to the cavity of outer ply. Upon measurement of elongation by pressurizing the cavity of outer ply, the outer ply may be detached from the blood vessel prosthesis so that the strength properties of the outer ply will not change or an outer ply having similar strength properties to those of the blood vessel prosthesis may be prepared alone and used.

When a pressure of 200 mmHg is applied to the inner cavity of itself and the inner cavity of its outer ply, assuming a and b refer to percentage increases in the inner diameters of the above cavities, respectively, and d refers to the inner diameter of non-pressurized state of the blood vessel prosthesis, the inner diameter of the blood vessel prosthesis of (1+a/100)d in the state of normal use may be expanded to the maximum (1+b/100)d (the thickness of the inner ply is negligibly small as compared with the inner diameter of the blood vessel prosthesis and the contribution by the intermediate ply in supporting blood is not taken into consideration after the separation) due to the separation between the inner ply and the intermediate ply with concomitant breakage of the intermediate ply. The degree of inflation in this case, i.e., an increase in the value of (1+b/100)d/((1+a/100)d) applies a large stress (strain) to the anastomotic part and there arises the risk of the diastasis of the anastomotic part.

Generally, even if there is a time-dependent dilation of about 20% after the implantation of a blood vessel prosthesis, they are believed there occurs substantially no trouble of the diastasis of the anatomotic part. Therefore, if the relationship (1+b/100)d/((1+a/100)d) ≤ 1.2, that is 1.2a+20 ≥ b is established, the risk of the diastasis of the anastomotic part accompanied by the inflation of blood vessel prosthesis can be minimized even if the separation between the inner ply and the intermediate ply should have occurred.

Furthermore, by making the value of a in the invention smaller, changes in diameter of blood vessel prosthesis before and after the recommencement of blood flow upon the implantation can be made smaller so that adaptation of size to that of the blood vessel of organism can be facilitated. However, if the value of a is too small, the blood vessel prosthesis becomes harder and is difficult to handle. In the present invention, a is preferably in the range of 5 to 40, more preferably 10 to 20.

As stated above, in the structure of the present invention, the inner ply material indirectly supports blood through intermediary of the adhesion to the intermediate ply. Therefore, the more the inner ply is involved in supporting blood, the more the risk of breakage of the adhesion layer, i.e., inter-ply separation between the inner ply and the intermediate ply. The technical idea of the present invention to minimize the change in inner diameter of blood vessel prosthesis upon loss of the supporting blood by the inner ply and intermediate ply simultaneously means minimizing from the beginning the participation of the inner ply and intermediate ply in supporting blood. That is, in the present invention, there occurs no large separation force between the inner ply and the intermediate ply, so that the inter-ply separation itself becomes difficult to occur. Therefore, the tubular blood vessel prosthesis of the present invention, composed of an outer ply and an inner ply, each being porous, and a nonporous intermediate ply is a blood vessel prosthesis that requires no preclotting, minimizes the possibility of inter-ply separation between the inner ply and the intermediate ply, and causes less risk of breakage or the diastasis of the anastomotic part if separation does occur at worst.

The force applied to the tubular blood vessel prosthesis by blood pressure is derived from the rule of Laplace:

$$T = (Pe-Pi)\gamma/\delta$$

(wherein T: tensile strength along the circumferential direction, (Pe-Pi): pressure difference between the outside and the inside (blood pressure), γ: inner diameter of the blood vessel prosthesis, δ: wall thickness of the blood vessel prosthesis). When calculation is performed using the above rule, in the state where the blood vessel prosthesis of 36 mm in inner diameter is pressurized at 200 mmHg, the blood vessel prosthesis is drawn by a force of about 50 gf per mm of width.

The production method for the blood vessel prosthesis of the present invention is not particularly limited. For example, use is made of (1) a method which comprises laminating an intermediate ply material in the form of a film on a surface of an inner ply, covering thereon an outer ply material, heat fusing the intermediate ply material to thermally fusing the three, (2) a method which comprises forming a layer of nonwoven fabric as an intermediate ply material on a surface of an inner ply material, covering thereon an outer ply material, then heat fusing the intermediate layer to render the intermediate ply material nonporous and simultaneously thermally fusing the three, and (3) a method which comprises covering an outer ply material with an intermediate ply material and then with an inner ply material in order and thermally fusing the three, reversing the tube inside out.

When these methods are used, use of crimpy fiber or shrinkable fiber in the outer ply (the inner ply in the case of (3) above) makes it easy to cover the intermediate ply therewith and subsequent heat-shrinking makes it to closely adhere to the intermediate ply, so that there is the advantage that the production can be performed without using a mandrel and the like. In the case where the intermediate ply is heat-molten and thermally fused, the heat-melting may be effected by the heating for shrinking the crimpy fiber or shrinkable fiber of the outer ply (the inner ply in the case of (3) above), or the heat-melting may be effected by providing another heating step after the heating for shrinking the crimpy fiber or shrinkable fiber.

The above method (3), makes the inner ply material originally present on the outside of the intermediate ply inside by reversion. This naturally gives more or less a surplus in the surface area of the cavity inner ply) of the blood vessel prosthesis. This can weaken the separation force between the inner ply and the intermediate ply. Note that similar effects can be obtained when the inner ply with a structure having a certain "allowance" is formed without using the method (3) above by, for example, a method of braiding at a tension lower than usual.

The present invention can also be practiced by a method which comprises directly winding the fiber of the outer ply around the intermediate ply. However, the method has the problem of balancing the penetrability of the tissue of organ and mechanical properties as compared with the above production method using knitted or woven texture.

The blood vessel prosthesis of the present inventin may be provided not only in the form of a straight tube but also in the form of a branched tube, or may be provided with crimp (bellows) and the like in order to impart resistance to kinking.

The blood vessel prosthesis of the present invention may be of 7 to 40 mm in inner diameter depending on the application site. In particular, when the inner diameter is 14 to 40mm, the blood vessel prosthesis can be used for substituting thoracic or abdominal aorta that has been difficult with the conventional blood vessel prostheses. When crimp is provided in the blood vessel prosthesis of the present invention, the inner diameter used in the present invention is defined as the inner diameter where the crimp is stretched.

In the blood vessel prosthesis of the present invention, the thickness of the outer ply is preferably 200 to 600 μm, more preferably 300 to 500 μm, the thickness of the intermediate ply is preferably 50 to 500 μm, more preferably 100 to 300 μm, and the thickness of the inner ply is preferably 100 to 500 μm, more preferably 200 to 400 μm.

As stated above, although the present invention has been described about embodiments of the present invention with reference to the attached drawings, the present invention should not be construed as being limited to the above embodiments but it should be noted that the invention may take various constitutions and various variations and modifications may be made within the scope of the invention.

EXAMPLES

Hereafter the presnet invention will be described in more detail by examples. However, the present invention is not limited thereto.

Example 1

A conjugated fiber (30 deniers, 18 filaments) of side by side type of polyethylene terephthalate/polybutylene terephthalate (1:1) was double russell-knitted to fabricate an inner ply material of a porous tubular form having an inner diameter of 35 mm, a knitting density of 71 loops/inch in warp, and 45 loops/inch in woof; a thickness of 0.44 mm, and an outer ply material of a porous tubular form having an inner diameter of 35 mm; a knittng density of 60 loops/inch in warp, and 58 loops/inch in woof; a thickness of 0.50 mm.

The inner ply material was covered on a TEFLON round rod of 28 mm in diameter, and immersed in hot water at 90°C for 10 minutes to introduce crimp to adhere it to the TEFLON round rod. After mixing styrene-ethylene-propylene-styrene (SEPS) block copolymer (SEPTON 4033, produced by Kuraray Co.) and squalane (Squalane-N, produced by Kuraray Co.) in a ratio of 1:1, the mixture was melt molded at 150°C to fabricate an intermediate ply material in the form of a film having a thickness of 0.20 mm and this was wound around the inner ply material. Further, the outer ply material was covered thereon and the heated at 90°C for 1 hour to introduce crimp to the outer ply material and at the same time melt the intermediate ply material to obtain a tubular blood vessel prosthesis having an inner diameter of 28 mm which has a structure composed of bonded three plies, i.e., a porous outer ply, a nonporous intermediate ply and a porous inner ply.

Example 2

By plain weaving polyethylene terephthalate fiber (45 deniers, 18 filaments) having a boiling water shrink ratio of 40%, an inner ply and an outer ply, each being porous and tubular, having an inner diameter of 35 mm and a water permeability of 100 ml/(min cm$^2$) were prepared. Using these elements and the intermediate ply material of Example 1, a tubular blood vessel prosthesis having an inner diameter of 28 mm which has a structure composed of bonded three plies, i.e., a porous outer ply, a nonporous intermediate ply and a porous inner ply was obtained in a manner similar to that in Example 1.

Comparative Example 1

A tubular blood vessel prosthesis having an inner diameter of 28 mm which has a structure composed of bonded three plies, i.e., a porous outer ply, a nonporous intermediate ply and a porous inner ply was obtained in a manner similar to that in Example 1 except that the inner ply material was used as an outer ply material and the outer ply material was used as an inner ply material.

Test Example 1

Relationship between the elongation of blood vessel prosthesis and its outer ply material
The cavities of the respective blood vessel prostheses obtained in Examples 1 and 2 and Comparative Example 1 were filled with water and an increase ratio of inner diameter (a%) when pressurized at 200 mmHg was measured. Separately, the outer ply of each of the blood vessel prostheses obtained in Examples 1 and 2 and Comparative Example 1 was removed and a rubber tube of 20 mm in inner diameter was inserted into the cavity of the outer ply and an increase in inner diameter (b%) when pressurized so that the pressure of 200 mmHg was applied was measured.
As a result of measurement, Example 1 showed a=30, b=43, Example 2 showed a=5, b=10, and Comparative Example 1 showed a=30, b=95. The values in Examples 1 and 2 satisfied the relationship 1.2a +20 ≥ b, and a ≤ 40 which is a characteristic of the blood vessel prosthesis of the present invention. On the contrary, the value in Comparative Example 1 satisfied the relationship a ≤ 40 but fails to satisfy the relationship 1.2a +20 ≥ b.
The inner ply and the intermediate ply in each of the blood vessel prostheses are bonded strongly and it was difficult to separate by hand.

Test Example 2 Pulsation test on blood vessel prostheses

To each of the blood vessel prostheses obtained in Examples 1 and 2 and Comparative Example 1 was filled physiological saline, and pulsation pressure was applied under the conditions of 60 cycles/min, maximum pressure of 200 mmHg, minimum pressure of 0 mmHg, and the maximum value of inner diameter was measured.

Thereafter, as simulation of the case where they are implanted in the body for a long period of time, the blood vessel prostheses were each immersed in 1N silver nitrate aqueous solution at 90°C for 1 month. In the respective blood vessel prostheses after the immersion, the adhesion between the inner ply and the intermediate ply was decreased so that they could be readily separated from each other by hand.

To these were again applied pulsation pressure under the same conditions as before treatment for 1 week and the maximum values of inner diameter were measured and changes in appearance were observed.

As a result of measurement, the blood vessel prosthesis of Example 1 showed no change in the appearance such as leakage or aneurysmal deformation and the inner diameter (maximum value) inflated slightly as compared with that before the immersion. The ratio of inflation was about 10%, which is far below 20% which indicates the fear of clinical problem. The blood vessel prosthesis of Example 2 showed no change in the appearance such as leakage or aneurysmal deformation and the inner diameter (maximum value) did not change at all as compared with that before the immersion.

On the contrary, the blood vessel prosthesis of Comparative Example 1 showed leakage and aneurysmal deformation from place to place after the immersion and depending the site, the aneurysmal deformation expanded to extend over the whole periphery of the blood vessel prosthesis, which indicates clear inflation. At the site where the inflation was greatest, the inner diameter (maximum value) was 50% in inflation ratio, which is above 20% that indicates the fear of clinical problem.

INDUSTRIAL APPLICABILITY

The blood vessel prosthesis of the present invention is of a laminate structure composed of three plies, i.e., an inner ply and an outer ply, each being porous, and a nonporous intermediate ply and it has the feature that it requires no preclotting and merges with the tissue of organism quickly after the implantation. The blood vessel prosthesis of the present invention minimizes the risk of inter-ply separation between the inner ply and the intermediate ply which would be potentially present in blood vessel prostheses having similar structures by controlling the relationship between the elongation properties of the blood vessel prosthesis itself and of the outer ply material within a certain range and minimizes the risk of the breakage of the prosthesis or the diastasis of the anastomotic part can be relieved, even if the separation should have occurred. The prosthesis is very useful since it is usable safely in the thoracic or abdominal cavity where no reinforcing effect by the surrounding tissues can be expected.

**Claims**

1. A tubular blood vessel prosthesis (1) comprising an outer ply (2) and an inner ply (4), each being biologically non-absorbable and porous, and a biologically non-absorbable and nonporous intermediate ply (3), the blood vessel prosthesis satisfying the relationships of $1.2a + 20 \geq b$ and $a \leq 40$ when a pressure of 200 mmHg is applied to the inner cavity of itself and the inner cavity of its outer ply, wherein a and b refer to percentage increases in the inner diameters of the above cavities, respectively.

2. A blood vessel prosthesis (1) as claimed in claim 1, wherein the inner diameter is 14 to 40 mm.

3. A blood vessel prosthesis (1) as claimed in claim 1 or 2, wherein the inner ply (4) and/or outer ply (2) are or is composed of knitted or woven texture of crimpy fiber.

4. A blood vessel prosthesis (1) as claimed in claim 1 or 2, wherein the inner ply (4) and/or outer ply (2) are or is composed of knitted or woven texture of shrunk fiber having a boiling water shrink ratio of 15 to 75%.

5. A blood vessel prosthesis (1) as claimed in claim 3, wherein the crimpy fiber is a conjugated fiber of polyethylene terephthalate and polybutylene terephthalate.

**Patentansprüche**

1. Rohrförmige Blutgefäßprothese (1) mit einer Außenlage (2) und einer Innenlage (4), die jeweils biologisch nicht absorbierbar und porös sind, und einer biologisch nicht absorbierbar und nicht porösen Zwischenlage (3), wobei die Blutgefäßprothese die Beziehungen $1{,}2a + 20 \geq b$ und $a \leq 40$ erfüllt, wenn ein Druck von 200 mmHg auf ihren inneren Hohlraum und den inneren Hohlraum ihrer Außenlage aufgebracht wird, wobei sich a und b jeweils auf Prozentsatzerhöhungen der Innendurchmesser der vorstehenden Hohlräume beziehen.

2. Blutgefäßprothese (1) nach Anspruch 1, wobei der Innendruchmesser 14 bis 40 mm beträgt.

**3.** Blutgefäßprothese (1) nach Anspruch 1 oder 2, wobei die Innenlage (4) und/oder die Außenlage (2) aus einer gestrickten oder gewebten Struktur aus einer gekräuselten Faser besteht/bestehen.

**4.** Blutgefäßprothese (1) nach Anspruch 1 oder 2, wobei die Innenlage (4) und/oder die Außenlage (2) aus einer gestrickten oder gewebten Struktur aus einer Schrumpffaser besteht/bestehen, die ein Siedewasserschrumpfverhältnis von 15 bis 75 % hat.

**5.** Blutgefäßprothese (1) nach Anspruch 3, wobei die gekräuselte Faser eine konjugierte Doppelbindungen enthaltende Faser aus Polyethylen-Terephthalat und Polybutylen-Terephthalat ist.

**Revendications**

**1.** Prothèse de vaisseau sanguin tubulaire (1) comprenant une couche externe (2) et une couche interne (4), chacune étant biologiquement non absorbable et poreuse, et une couche intermédiaire (3) biologiquement non absorbable et non poreuse, la prothèse de vaisseau sanguin satisfaisant les relations $1,2a+20 \geq b$ et $a \leq 40$ quand une pression de 200 mm Hg est appliquée à sa cavité interne et à la cavité interne de sa couche externe, où a et b désignent les augmentations en pourcentage des diamètres internes des cavités ci-dessus, respectivement.

**2.** Prothèse de vaisseau sanguin (1) selon la revendication 1 où le diamètre interne est 14 à 40 mm.

**3.** Prothèse de vaisseau sanguin (1) selon la revendication 1 ou 2 où la couche interne (4) et/ou la couche externe (2) est ou sont composées de texture tricotée ou tissée de fibres frisées.

**4.** Prothèse de vaisseau sanguin (1) selon la revendication 1 ou 2 où la couche interne (4) et/ou la couche externe (2) est ou sont composées de texture tricotée ou tissée de fibres rétrécies ayant un taux de retrait dans l'eau bouillante de 15 à 75 %.

**5.** Prothèse de vaisseau sanguin (1) selon la revendication 3 où les fibres frisées sont des fibres conjuguées de polyéthylènetéréphtalate et de polybutylènetéréphtalate.

FIG.1

# FIG.2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5131025 A
- JP 7051354 A
- US 4743252 A
- JP 8332218 A
- US 5354329 A